# EUROPEAN PATENT APPLICATION

(11) **EP 2 818 100 A1**
(43) Date of publication of application: **31.12.2014**
(21) Application number: 14168647.7
(22) Date of filing: 16.05.2014
(51) Int. Cl.: A61B 5/00

(54) **Method for analyzing tissue cells using hyperspectral imaging**

(30) Priority: 27.06.2013 TW 102122922
(71) Applicant: China Medical University, Taichung City 404 (TW)
(72) Inventor: Duann, Jeng-Ren, 404 North Dist., Taichung City (TW); Chiou, Jin-Chern, 404 North Dist.,Taichung City (TW); Lin, Yung-Jiun, 404 North Dist.,Taichung City (TW); Tsai, Ming-Hsui, 404 North Dist., Taichung City (TW)
(74) Representative: 2K Patentanwälte Blasberg Kewitz & Reichel

(57) **Abstract**

A method and system are used to analyze the components of a tissue sample (30) by using hyperspectral imaging. The system comprises an image capture module (10) and a hyperspectral image analysis module (20). The image capture module (10) generates an excitation light beam to illuminate the tissue sample (30), receives a spectral image induced by the excitation light beam, and converts the spectral image into hyperspectral image data (121) containing continuous spectrum waveforms. The hyperspectral image analysis module (20) performs a linear transformation on the hyperspectral image data (121) to obtain a plurality of linearly-independent continuous spectrum curves and compares the linearly-independent continuous spectrum curves with continuous spectrum data of known components in a database (23) to identify the components in the tissue sample (30) and obtain the types, proportions, and spatial distributions thereof, whereby the physician can diagnose the lesion more accurately.

## Description

### FIELD OF THE INVENTION

The present invention relates to a method for analyzing tissue cells, particularly to a hyperspectral imaging method for analyzing tissue cells.

### BACKGROUND OF THE INVENTION

Early detection of cancer is known to increase the cure rate of cancer patients. How to fast and effectively detect cancers at an early stage has been the common problem the researchers in the medical field desire to overcome. The current cancer screening system uses biopsy to identify and stage a cancer. The biopsy is using a microscope to examine whether the tissue has cells differentiating abnormally. The cells cancered seriously can be easily recognized. However, some early-stage disorders have so diversified appearances that even for experienced physicians it is difficult or even impossible to identify them.

Some fluorescent detection systems were developed to recognize abnormal cells in tissue. A US patent of publication No.20050272027 disclosed "Real-Time Clinical Diagnostic Expert Systems for Fluorescent Spectrum Analysis of Tissue Cells and Methods Thereof", which comprises steps: using excitation light to illuminate a specified epidermis tissue and make the epidermis tissue generate an autofluorescent signal; capturing and analyzing the continuous spectrum of the autofluorescent signal; undertaking comparisons of the slopes, intensities and peaks in a specified range of the spectrum; and establishing a weight table to increase the identification accuracy. In fact, the spectrum detected at an identical point has many spectral signals coming from different components. It means that the detected spectrum is a combination of autofluorescent signals of many components. The components may be molecular-scale chemical compounds, proteins, DNA, RNA, or cell nuclei containing various materials. Interference between different components may impair the accuracy of test. Besides, the prior art cannot precisely determine the exact locations of inflammatory tissues or cancerous tissues but can only determine whether there is an inflammatory tissue or a cancerous tissue. Therefore, the conventional technology still has much room to improve.

### SUMMARY OF THE INVENTION

The primary objective of the present invention is to improve the accuracy of a hyperspectral imaging system in identifying components of tissue.

Another objective of the present invention is to solve the problem that the conventional technology cannot determine the positions and distribution of abnormal cells.

To achieve the abovementioned objectives, the present invention proposes a hyperspectral imaging system for analyzing tissue cells, which is used to identify components in a tissue sample and obtain distributions of the components in the tissue sample, and which comprises an image capture module and a hyperspectral image analysis module.

The image capture module includes an optical detection unit and a spectrum conversion unit. The optical detection unit has a light source generating excitation light to illuminate a tissue sample and a spectral image detector receiving the spectral image generated by the tissue sample. The spectrum conversion unit receives the signal output by the spectral image detector and converts the signals into hyperspectral image data containing continuous spectrum waveform signals. The hyperspectral image analysis module includes a linear transformation unit, a database, and a comparison unit connected with the linear transformation unit and the database. The database stores continuous spectrum data of a plurality of known components. The linear transformation unit respectively calculates the continuous spectrum waveform signals of the hyperspectral image data to obtain a plurality of linearly-independent continuous spectrum curves and the proportions thereof. The comparison unit compares the continuous spectrum curves with the continuous spectrum data of known components in the database to determine the components in the tissue sample, the types thereof and the proportions thereof.

The present invention also proposes a hyperspectral imaging method for analyzing tissue cells, which comprises
Step S 1: obtaining a tissue sample;
Step S2: using a first excitation light beam of an optical detection to illuminate the tissue sample to make the tissue sample generate a spectral image;
Step S3: using a spectrum conversion unit to convert the spectral image into hyperspectral image data containing continuous spectrum waveform signals;
Step S4: performing a linear transformation on the hyperspectral image data to calculate a plurality of linearly-independent continuous spectrum curves and the proportions thereof; and
Step S5: using a comparison unit to compare the continuous spectrum curves with the continuous spectrum data of known components in a database to determine the components in the tissue sample, the types thereof and the proportions thereof.

The present invention is characterized in
1. Capturing continuous spectral image generated by the tissue sample to obtain hyperspectral image data and preserving the waveform characteristics of the continuous spectrum curves of the entire spectral image;
2. Using a linear transformation to analyze the hyperspectral image data to extract linearly-independent continuous spectrum curves from the hyperspectral imaging data, and comparing the linearly-independent spectrum curves with the spectrum curve data of known components in the database;
3. Not using the peaks of a specified frequency band to determine the component in a tissue sample, but using the waveform characteristics of an entire continuous spectrum curve to determine a component, the type thereof and the proportion thereof, whereby to reduce the probability of false identification;
4. Comparing the linearly-independent continuous spectrum curves to obtain quantified waveforms and the component proportion indexes to function as hyperspectral biomarkers.

### BRIEF DESCRIPTION OF THE DRAWINGS

Fig.1 schematically shows the architecture of a hyperspectral imaging system for analyzing tissue cells according to one embodiment of the present invention;
Fig.2 is a flowchart of a method according to one embodiment of the present invention;
Fig.3A schematically shows the linear transformation of continuous spectrum data according to a first embodiment of the present invention;
Fig.3B schematically shows the linear transformation of continuous spectrum data according to a second embodiment of the present invention; and
Fig.4 schematically shows the distribution of a component according to one embodiment of the present invention.

### DETAILED DESCRIPTION OF THE PREFERRED EMBODIMENTS

The technical contents of the present invention are described in detail in cooperation with drawings below.

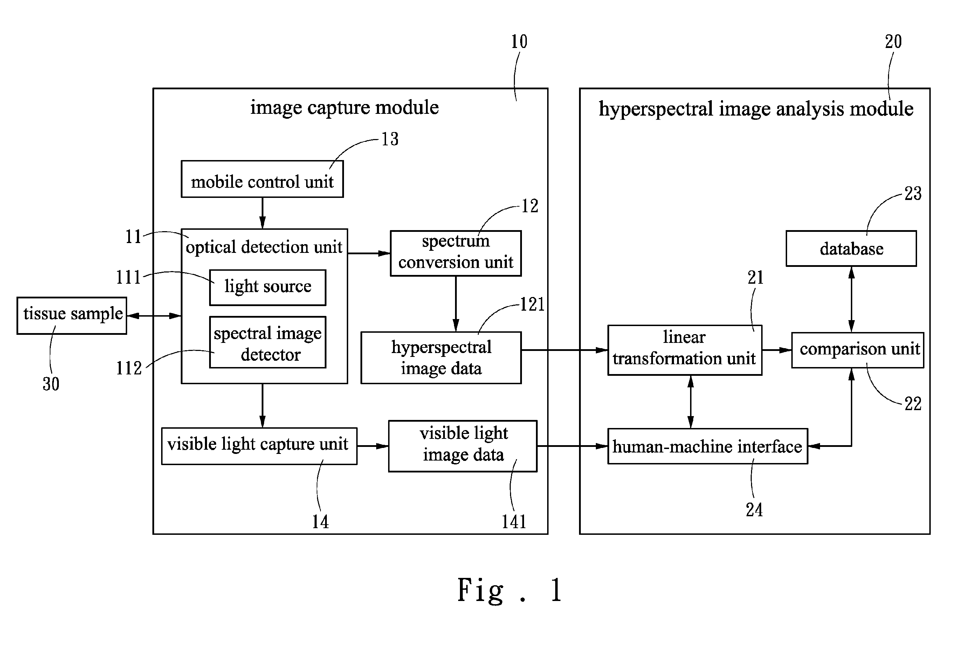

Refer to Fig.1. The present invention proposes a hyperspectral imaging system for analyzing tissue cells, which is used to analyze the components of a tissue sample 30, and which comprises an image capture module 10 and a hyperspectral image analysis module 20. The image capture module 10 includes an optical detection unit 11 and a spectrum conversion unit 12. The optical detection unit 11 has a light source 111 generating an excitation light beam to illuminate the tissue sample 30 and a spectral image detector 112 receiving the spectral image generated by the tissue sample 30. The spectral image is an autofluorescent image or an absorption spectrum image. The autofluorescent image is the fluorescent image emitted by the tissue sample 30 having received the excitation light generated by the light source 111. The absorption spectrum image is the spectrum where a portion of frequency bands of the light source 111 have been absorbed by the tissue sample 30. The spectrum conversion unit 12 receives the signals output by the spectral image detector 112 and converts the signals into hyperspectral image data 121 containing continuous spectrum waveform signals. The hyperspectral image analysis module 20 includes a linear transformation unit 21, a database 23, and a comparison unit 22 connected with the linear transformation unit 21 and the database 23. The database 23 stores the continuous spectrum data of a plurality of known components. The linear transformation unit 21 calculates the continuous spectrum waveform signals of the hyperspectral image data 121 to obtain a plurality of linearly-independent continuous spectrum curves and the proportions thereof. The comparison unit 22 compares the linearly-independent continuous spectrum curves with the continuous spectrum data of known components in the database 23 to determine the components in the tissue sample 30, the types thereof and the proportions thereof. In this embodiment, the user uses the system to analyze the components of the tissue sample 30 and thus learns the positions of normal cells and cancered cells. Via the proportions of the components, the user can further learn the cancer stage. Although some independent continuous spectrum curves may come from the unknown components of the tissue sample 30, some may be the noise introduced during the test. The way to exclude the noise from the received signals will be described thereinafter.

In one embodiment, the image capture module 10 also includes a mobile control unit 13 used to undertake 2D (2-Dimensional) scans on the tissue sample 30, whereby the hyperspectral image data 121 contains longitudinal-axis positional signals, transverse-axis positional signals and continuous spectrum waveform signals, which thus form 3D (3-Dimensional) hyperspectral image data 121. In other words, the hyperspectral image data 121 consists of the information obtained at a plurality of detected points. The continuous spectrum waveform signal obtained at each individual detected point is not a single value but a continuous spectrum curve recording different intensities corresponding to different wavelengths. The linear transformation unit 21 may use but is not limited to use ICA (Independent Component Analysis), PCA (Principal Component Analysis) or factor analysis to undertake linear transformation and analysis. The linear transformation unit 21 undertakes linear transformations on the continuous spectrum curves of a plurality of detected points, which are contained in the hyperspectral image data 121, at the same time to obtain a plurality of linearly-independent continuous spectrum curves of each detected point.

In one embodiment, the hyperspectral image analysis module 20 also includes a human-machine interface 24 connected with the linear transformation unit 21 and comparison unit 22. According to the component comparison results of the comparison unit 22, the human-machine interface 24 outputs a graphical image showing the components of each detected point on the tissue sample 30. Further, the human-machine interface 24 uses different colors to denote different components and uses the gray level to denote the proportion of each component. The user operates the human-machine interface 24 to start or control the system. The user may further use the human-machine interface 24 to rotate the image and magnify a specified area of the image. Thereby, the user can learn the components of the tissue sample 30 more instinctively. In one embodiment, the image capture module 10 also includes a visible light capture unit 14 connected with the optical detection unit 11 and used to obtain visible light image data 141. The visible light image data 141 presents the image of the appearance of the tissue sample 30 the same as the human eye sees, including the derma, the epidermis, and other structures of the tissue sample 30. The visible light capture unit 14 is also connected with the human-machine interface 24. The human-machine interface 24 superimposes the visible light image data 141 on the graphic image to form graphic data. In other words, the component data of the graphic image is directly superimposed on the visible light image data 141, whereby the user can learn the components and the proportions of the components of each region on the tissue sample 30 more instinctively.

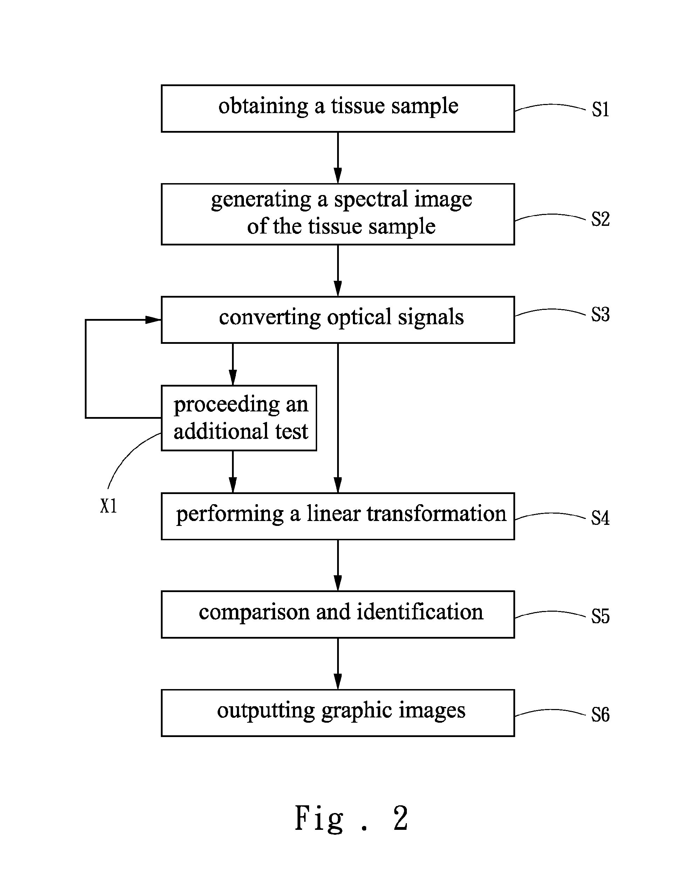

Refer to Fig.2. The present invention also proposes a hyperspectral imaging method for analyzing tissue cells, which comprises Steps S1-S6.

Step S1: obtaining a tissue sample 30. The tissue sample 30 may be acquired via biopsy. Alternatively, the image capture module 10 directly captures the image of the lesion.

Step S2: generating a spectral image of the tissue sample 30. In one embodiment, the optical detection unit 11 uses a first excitation light beam to illuminate the tissue sample 30 and make the tissue sample 30 generate a spectral image. In one embodiment, a light source 111 having wavelengths of 330-385nm is used to illuminate the tissue sample 30 and make the tissue sample 30 generate a spectral image. The spectral image is an autofluorescent image or an absorption spectrum image. The autofluorescent image is the fluorescent image emitted by the tissue sample 30 having received the excitation light generated by the light source 111. The absorption spectrum image is the spectrum where a portion of frequency bands of the light source 111 have been absorbed by the tissue sample 30.

Step S3: converting optical signals. The spectrum conversion unit 12 converts the spectral image into hyperspectral image data 121 containing a continuous spectrum waveform signal. In one embodiment, the spectrum conversion unit 12 uses a mobile control unit 13 to scan detected points of the tissue sample 30, whereby the hyperspectral image data 121 contains longitudinal-axis positional signals X, transverse-axis positional signals Y and continuous spectrum waveform signals λ, which thus form 3D hyperspectral image data 121. For example, the user sets an interesting point as the center of a detected region and uses 500 points on the transverse axis and 500 point on the longitudinal axis to divide the detected region into 250000 detected points each corresponding to a continuous spectrum waveform signal. In one embodiment, the frequency band ranging from 400nm to 1000nm is divided by 3nm to have 200 frequency coordinate points. Thus, the hyperspectral image data of the detected region is 3D information denoted by [X, Y, λ] with a resolution of [500, 500, 200]. The detected region and the resolution can be adjusted to meet requirement.

In order to detect the components in further detail or detect the detected region with a second excitation light beam, the present invention arranges Step X1 succeeding to Step S3. In Step X1, the optical detection unit 11 uses a second excitation light beam to illuminate the tissue sample 30 so as to generate a corresponding spectral image. Then, the process returns to Step S3 to perform optical signal conversion. Thereby is obtained another type of fluorescent spectrum information and achieved a further complete identification. In one embodiment, a second excitation light beam having wavelengths ranging from 470nm to 490nm is used to illuminate the tissue sample so as to obtain the hyperspectral image data of second spectral images.

Step S4: performing a linear transformation. Perform a linear transformation on the hyperspectral image data 121 to convert the continuous spectrum waveform signals of the detected points into linearly-independent continuous spectrum curves and the proportions thereof. The linear transformation may be realized with the ICA method, the PCA method or the factor analysis method. In the present invention, the linear transformation is realized with the ICA method. After ICA, the continuous spectrum curve of each detected point is decomposed to discriminate different components existing in the detected point. The ICA method can exclude the noise affection and exempt the analysis from noise interference. As the ICA method is not the focus of the present invention, it will not be described in detail herein.

Step S5: comparison and identification. A comparison unit 22 is used to compare each linearly-independent continuous spectrum curve with the continuous spectrum curves of known components in the database 23 to identify the components of the tissue sample 30 and obtain the proportions thereof. As the present invention can undertake planar scanning, the component distributions in the tissue sample 30 can be obtained simultaneously.

Step S6: outputting graphic images. A human-machine interface 24 outputs a graphic image according to the comparison result. The user can operate the human-machine interface 24 to present the graphic image of a specified position, whereby the user can learn the components existing in a specified position and the proportion thereof. Then, the user may use the pathological knowledge to determine the cancer stage of abnormal cells.

For example, abnormal keratin hyperplasia occurs in the basal lamina of the epithelium during the differentiation process of oral cancer. The present invention can determine the position and proportion of keratin hyperplasia and provide precisely quantified information about the proportion of keratin hyperplasia to epithelium and the distribution of abnormal keratin hyperplasia for quantifying the cancer stage.

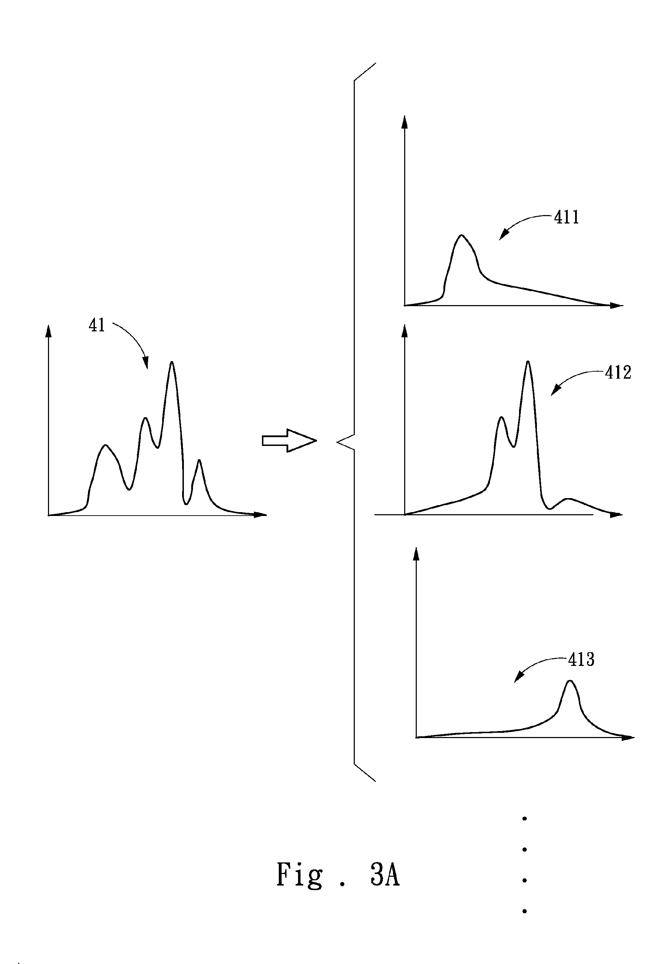

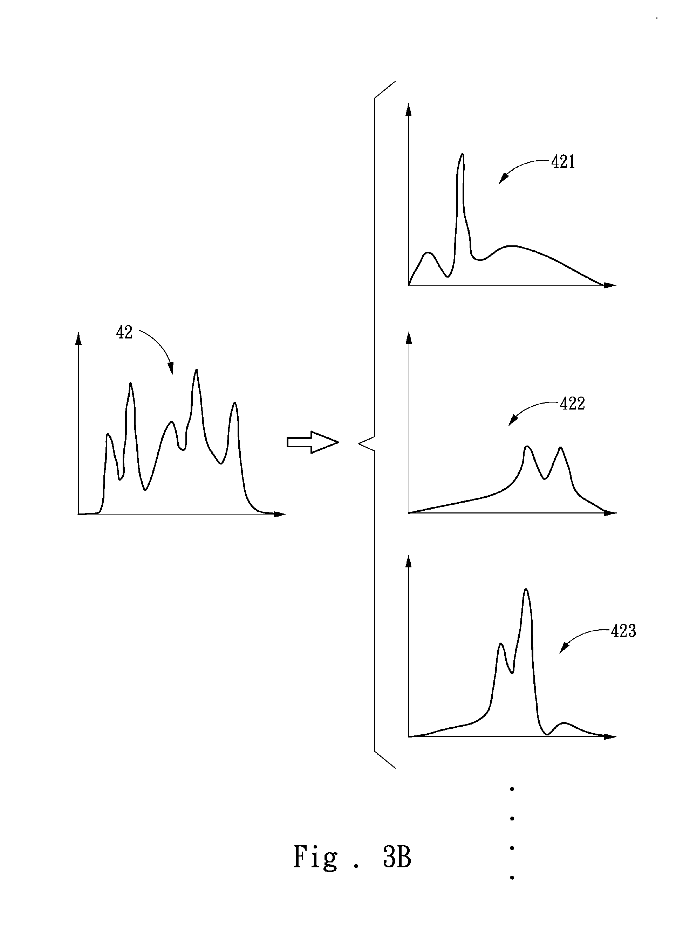

Refer to Fig.3A and Fig.3B respectively showing a first continuous spectrum curve 41 and a second continuous spectrum curve 42, which are separately converted from the spectral images of two different detected points of the tissue sample 30. The different spectral images of the two different detected points are converted into different continuous spectrum curves 41 and 42. Refer to Fig.3A. The linear transformation unit 21 of the hyperspectral image analysis module 20 undertakes a linear transformation to divide the first continuous spectrum curve 41 into a first linearly-independent continuous curve 411, a second linearly-independent continuous curve 412, and a third linearly-independent continuous curve 413, and each linearly-independent continuous curve represents a component. The present invention does not identify a component with a single wavelength but identifies a component with the profile of the entire curve, such as the first linearly-independent continuous curve 411, the second linearly-independent continuous curve 412, and the third linearly-independent continuous curve 413. Thereby is reduced the probability of false identification.

Refer to Fig.3B. The linear transformation unit 21 of the hyperspectral image analysis module 20 undertakes a linear transformation to divide the second continuous spectrum curve 42 into a fourth linearly-independent continuous curve 421, a fifth linearly-independent continuous curve 422, and a sixth linearly-independent continuous curve 423, and each linearly-independent continuous curve represents a component. The database 23 stores the continuous spectrum curves of various components. Therefore, the comparison unit 22 can identify the components corresponding to the first linearly-independent continuous curve 411, the second linearly-independent continuous curve 412, the third linearly-independent continuous curve 413, the fourth linearly-independent continuous curve 421, the fifth linearly-independent continuous curve 422, and the sixth linearly-independent continuous curve 423.

The second linearly-independent continuous curve 412 of the first continuous spectrum curve 41 and the sixth linearly-independent continuous curve 423 of the second continuous spectrum curve 42 have almost identical profiles, which indicates that the detected points corresponding to the first continuous spectrum curve 41 and the first continuous spectrum curve 41 have an identical component. The proportions of the component in the two detected points can be worked out from the proportion of the second linearly-independent continuous curve 412 in the first continuous spectrum curve 41 and the proportion of the sixth linearly-independent continuous curve 423 in the second continuous spectrum curve 42.

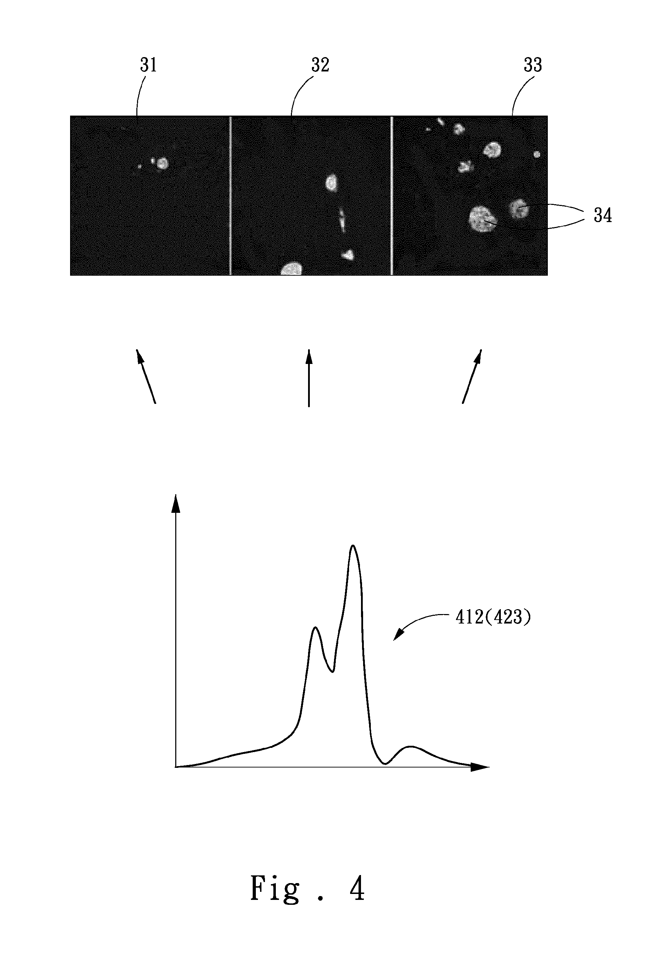

Refer to Fig.4. The regions containing the component corresponding to the second linearly-independent continuous curve 412 are labeled by white areas 34 in a first tissue sample 31, a second tissue sample 32 and a third tissue sample 33. If that the second linearly-independent continuous curve 412 is identical to the continuous spectrum curve of a specific component of a known cancer, which has been stored in the database 23, the white regions 34 are determined to be the positions of the cancer in the first tissue sample 31, the second tissue sample 32 and the third tissue sample 33. Further, the white regions 34 may be superimposed on the graphic images on the human-machine interface 24, whereby the physician can evaluate the test result more easily. In this embodiment, the regions containing the specific component are labeled by the white areas 34. In fact, the regions containing different components may be respectively labeled by different colors to present test results more clearly.

In conclusion, the present invention proposes a hyperspectral imaging method for analyzing tissue cells, which is characterized in
1. Picking up the continuous spectra emitted by all detected points of a tissue sample to obtain the hyperspectral imaging data and preserve the characteristics of the continuous spectrum curves of the entire spectral images;
2. Using linear a transformation to analyze the hyperspectral image data to extract linearly-independent continuous spectrum curves from the hyperspectral imaging data, and comparing the linearly-independent spectrum curves with the spectrum curve data of known components in the database;
3. Not using the peaks of a specified frequency band to determine a component in a tissue sample, but using the waveform characteristics of an entire continuous spectrum curve to determine a component, the type, proportion and distribution of the component, the positions of cancered cells and the cancer stage, whereby to reduce the probability of false identification;
4. Comparing the linearly-independent continuous spectrum curves to obtain quantified waveforms and the component proportion indexes to function as hyperspectral biomarkers;
5. Using the ICA method to obtain the waveforms of the linearly-independent continuous spectrum curves to effectively identify components and obtain the proportions thereof, whereby is obtained the quantified component proportions; and
6. Using the human-machine interface to superimpose graphic images on visible light images to enable the user to examine the components of the detected points and the proportions thereof more instinctively.

## Claims

1. A method for analyzing tissue cells using hyperspectral imaging, the method comprising:
obtaining a tissue sample (30) (S1);
using a first excitation light beam of an optical detection unit (11) to illuminate the tissue sample (30) and enable the tissue sample (30) to generate a spectral image (S2);
converting the spectral image by a spectrum conversion unit (12) into hyperspectral image data (121) containing a continuous spectrum waveform signal (S3);
performing a linear transformation on the hyperspectral image data (121) to calculate a plurality of linearly-independent continuous spectrum curves and proportions of the linearly-independent continuous spectrum curves (S4); and
using a comparison unit (22) to compare the linearly-independent continuous spectrum curves with continuous spectrum data of a plurality of known components in a database (23) to identify components in the tissue sample (30) and obtain types of the components and proportions of the components (S5).

2. The method according to claim 1, wherein in the step of converting the spectral image into hyperspectral image data (S3), a mobile control unit (13) performs 2-dimensional scanning on the tissue sample (30) to make the hyperspectral image data (121) contain longitudinal-axis positional signals, transverse-axis positional signals and the continuous spectrum waveform signals and to form 3-dimensional hyperspectral image data (121) facilitating to obtain spatial distributions of the components in the tissue sample (30) in the succeeding steps.

3. The method according to claim 2 further comprising a step of outputting a graphic image (S6), in which a human-machine interface (24) outputts a graphic image according to the components performed by the comparison unit (22), wherein said step of outputting said graphic image (S6) succeeds said step S5 of comparing the linearly-independent continuous spectrum curves to identify components in the tissue sample (30) and obtain types of the components and proportions of the components.

4. The method according to claim 3, wherein in said step of outputting said graphic image (S6) a visible light capture unit (14) obtains visible light image data (141), and the visible light image data (141) is superimposed on the graphic image to form graphic data.

5. The method according to any of the preceding claims, wherein in said step S4 of performing a linear transformation on the hyperspectral image data, the linear transformation is an ICA (Independent Component Analysis) method, a PCA (Principal Component Analysis) method or a factor analysis method.

6. The method according to any of the preceding claims, further comprising Step X1: the optical detection unit (11) using a second excitation light beam to illuminate the tissue sample (30) so as to generate a corresponding spectral image, wherein Step X1 succeeds to step S3 of converting the spectral image into hyperspectral image data, and wherein after Step X1, the process returns to said step S3 of converting the spectral image into hyperspectral image data to perform optical signal conversion.

7. The method according to claim 6, wherein the spectral image is an autofluorescent image or an absorption spectrum image.
